(19) Europäisches Patentamt
European Patent Office
Office européen des brevets

(11) Veröffentlichungsnummer : **0 061 055**
**B1**

(12) **EUROPÄISCHE PATENTSCHRIFT**

(45) Veröffentlichungstag der Patentschrift : **23.05.84**

(51) Int. Cl.³ : **C 07 D493/04** // (C07D493/04, 307/00, 307/00)

(21) Anmeldenummer : **82101805.8**

(22) Anmeldetag : **08.03.82**

(54) **Verfahren zur Herstellung von 1.4-3.6-Dianhydro-hexitolen.**

(30) Priorität : **20.03.81 DE 3111092**

(43) Veröffentlichungstag der Anmeldung : **29.09.82 Patentblatt 82/39**

(45) Bekanntmachung des Hinweises auf die Patenterteilung : **23.05.84 Patentblatt 84/21**

(84) Benannte Vertragsstaaten : **BE DE FR GB IT**

(56) Entgegenhaltungen :
**JOURNAL OF THE CHEMICAL SOCIETY, 1947 LONDON (GB) R. MONTGOMERY: "Anhydrides of Polyhydric Alcohols. Part VI. 1 : 4-3 : 6-Dianhydro Mannitol and 1 : 4-3 : 6-Dianhydro Sorbitol from Sucrose" Seiten 433-436**

(73) Patentinhaber : **BAYER AG**
**Konzernverwaltung RP Patentabteilung**
**D-5090 Leverkusen 1 Bayerwerk (DE)**

(72) Erfinder : **Salzburg, Herbert, Dr.**
**Hahnenweg 3**
**D-5000 Köln 80 (DE)**
Erfinder : **Meyborg, Holger, Dr.**
**Bergisch-Gladbacher-Strasse 1**
**D-5068 Odenthal (DE)**
Erfinder : **Ziemann, Heinz, Dr.**
**Am Wiesenberg 10**
**D-5653 Leichlingen (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 061 055 B1

**Beschreibung**

Die Erfindung beschreibt ein verbessertes Verfahren zur Herstellung von 1.4-3.6-Dianhydro-hexitolen aus Hexitolen durch intramolekulare Wasserabspaltung mittels gasförmigen Chlorwasserstoffs, gegebenenfalls in Gegenwart von organischen Carbonsäuren, Carbonsäurechloriden und/oder Carbonsäureanhydriden, in Abwesenheit von Lösungsmitteln und/oder Wasser.

Die Verbindungsklasse der 1.4-3.6-Dianhydro-hexitole ist seit ca. 1880 bekannt und wird in folgenden Literaturstellen beschrieben :

1. Fauconier, Bull. Soc. Chem. (1884), *41,* 119 ;
2. L. F. Wiggins, J. Chem. Soc., *1945,* 4 ;
3. Haworth, Heath, Wiggins, J. Chem. Soc., *1944,* 155 ;
4. R. Montgomery, L. F. Wiggins, J. Chem. Soc., *1947,* 5 ;
5. J. C. Goodwin, J. E. Hodge, D. Weisleder, Carbohyd. Res., *79,* 133/1980).

Den Synthesen ist gemeinsam, daß alle in Gegenwart von Lösungsmitteln durchgeführt werden, wobei die intramolekulare Wasserabspaltung aus den Hexitolen durch saure Substanzen bewirkt wird. Die ebenfalls in der Literatur bekannten (lösungsmittelfreien) Synthesen bedienen sich schwerflüchtiger Säuren, z. B. Schwefelsäure, niemals aber Chlorwasserstoffgases. Die folgende Tabelle zeigt eine repräsentative Zusammenstellung von Lösungsmitteln bzw. Katalysatoren, weiterhin ist die Leistungsfähigkeit der Methode am im allgemeinen schlecht zugänglichen 1.4-3.6-Dianhydromannit illustriert :

| Lösungsmittel | Ausbeute an 1.4-3.6-Dianhydromannit (Isomannid)* | Literatur |
|---|---|---|
| Dichlorglycerin | ca. 36 % | 2) |
| konz. Salzsäure | 35 % | 3) |
| konz. Salzsäure | 40 % | 3) |
| konz. Salzsäure | 25 % | 4) |
| Essigester/Dioxan | = 35 % | 5) |

* Isomannid ist der in der Literatur gebräuchliche Name für 1.4-3.6-Dianhydro-D-mannit.

Der grundlegende Nachteil dieser Verfahren ist zum einen in der unbefriedigenden Ausbeute bzw. einem entsprechend großen Anfall an harzähnlichen Destillationsrückständen und zum anderen in der häufig erforderlichen Reaktionszeit (bis 85 Stunden) begründet. Diese Nachteile schließen eine Herstellung von z. B. Isomannid oder Isosorbid im großtechnischen Maßstab aus, zumal auch die Rohstoffpreise für die Ausgangsverbindungen Mannit und Sorbit, gemessen an den Preisen üblicher Ausgangschemikalien der chemischen Industrie, nicht unerheblich sind.

Aufgabe der Erfindung war es deshalb, die Herstellung von Di-anhydro-hexitolen (« Isohexitolen ») aus Hexitolen zu verbessern. Insbesondere sollte die Ausbeute wesentlich über die der bekannten Verfahren gesteigert werden, um eine wirtschaftlich vertretbare Produktion von Di-anhydro-hexitolen im industriellen Maßstab zu ermöglichen.

Di-anhydro-hexitole = (Di-anhydro-hexite)
« Isohexitole »

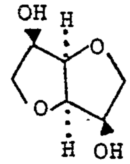

1.4-3.6-Dianhydro-D-mannit
« Isomannid »

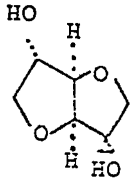

1.4-3.6-Di-anhydro-D-sorbit
« Isosorbid »

Überraschenderweise wurde nun in dem hier beschriebenen Verfahren gefunden, daß in einer unerwartet selektiven Reaktion aus Hexitolen die Isohexitole in guten Ausbeuten zugänglich sind, wenn auf die Verwendung von Lösungsmitteln verzichtet und gasförmiger Halogenwasserstoff, vorzugsweise Chlorwasserstoff, mit dem kristallinen oder sirupösen Hexitol zur Reaktion gebracht wird. Die Reaktion

kann sowohl bei Normaldruck wie auch vorzugsweise bei erhöhtem Druck durchgeführt werden, wobei die Reaktionspartner bei einer Temperatur von 0 bis 300 °C, vorzugsweise 20 bis 160 °C, besonders bevorzugt 30 bis 140 °C, und einem Druck von 1 bis 250 bar, besonders bevorzugt von 1 bis 40 bar, zur Reaktion gebracht werden. Es ist gleichfalls möglich, andere Halogenwasserstoffe wie HBr und HF zur Wasserabspaltung zu benutzen ; günstige Ausbeute liefert dabei auch HBr.

Besonders günstig wirkt sich auf die Selektivität der Wasserabspaltungsreaktion die Anwesenheit von organischen Carbonsäuren, Carbonsäurechloriden und symmetrischen sowie unsymmetrischen Carbonsäureanhydriden aus. Die eingesetzten Mengen an diesen zusätzlichen Verbindungen sind in weiten Grenzen variabel und betragen 0,01 bis 600 Mol-%, vorzugsweise 0,5 bis 10 Mol-%, besonders bevorzugt 1 bis 4 Mol-%, bezogen auf die Hexitole.

Wird ein Säurechlorid verwendet, so erübrigt sich gegebenenfalls die Zugabe von HCl, wenn sinngemäß durch Reaktion des Säurechlorids mit den OH-Gruppen das Hexitols die entsprechende Menge Chlorwasserstoff freigesetzt wird.

Vorzugsweise werden Carbonsäuren der allgemeinen Formel R—$(ROOH)_x$ mit x = 1 bis 6, wobei R ein x-wertiger Rest einer aliphatischen gesättigten oder ungesättigten, einer aromatischen oder araliphatischen Carbonsäure eingesetzt, z. B. von Ameisensäure, Essigsäure, Adipinsäure, Bernsteinsäure, Oxalsäure, Maleinsäure, Phthalsäure, Benzoesäure, Phenylessigsäure, Chloressigsäure, Trifluoressigsäure, ferner Kohlensäure, eingesetzt.

Als Säurehalogenide kommen die Halogenide der oben erwähnten Carbonsäuren der allgemeinen Formel

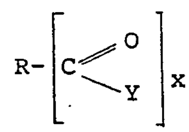

wobei

Y = Halogen, wie z. B. Chlor oder Brom ist und

x und R die oben angegebene Bedeutung haben.

Beispiele sind Acetylchlorid, Adipoylchlorid, Hexanoylchlorid, Oxalylchlorid, Benzoylchlorid, Phenylacetylchlorid.

Als Säureanhydride kommen symmetrische oder unsymmetrische Carbonsäureanhydride der allgemeinen Formel in Betracht, wobei X = 1 bis 6 und R gleiche oder verschiedene Reste der oben angeführten Bedeutung für R bedeuten. Beispiele sind Acetanhydrid, Bernsteinsäureanhydrid, Adipinsäurepolyanhydrid, Phthalsäureanhydrid und ähnliche Verbindungen.

Gegenstand der Erfindung ist somit ein Verfahren zur Herstellung von 1.4-3.6-Dianhydro-hexitolen, dadurch gekennzeichnet, daß man als saures Dehydratisierungsreagens gasförmigen Halogenwasserstoff und gegebenenfalls organische Carbonsäuren, Carbonsäure-halogenide und/oder Carbonsäureanhydride verwendet und die Reaktion in Abwesenheit von Wasser und organischen Lösungsmitteln durchführt.

Die wasserabspaltende Reaktion wird nach dem erfindungsgemäßen Verfahren dadurch realisiert, daß man in das Hexitol als Feststoff oder Flüssigkeit oder Schmelze den gasförmigen Chlorwasserstoff in 0,01 bis etwa 1 000-fachem äquivalentem Verhältnis, oder aber bis zur Sättigung bei der entsprechenden Temperatur und dem entsprechenden Druck einleitet, vorzugsweise in äquivalentem bis etwa 1 000-fach äquivalentem Verhältnis. Vorzugsweise werden die Hexitole im Gemisch mit organischen Carbonsäuren, Carbonsäurechloriden- und symmetrischen sowie unsymmetrischen Carbonsäureanhydriden in einem Verhältnis von 0,01 bis 600 Mol-%, vorzugsweise 0,5 bis 10 Mol-%, besonders bevorzugt 1 bis 4 Mol-%, bezogen auf Hexitole, versetzt und dann der Chlorwasserstoff beaufschlagt.

Zur Isolierung der Isohexitole kann entweder fraktioniert destilliert werden, wobei unter gleichzeitiger Zuendeführung der Reaktion Wasser und zusätzliche organische Carbonsäuren, Carbonsäurechloride und symmetrische sowie unsymmetrische Carbonsäureanhydride, anschließend im Wasserstrahl- bzw. Hochvakuum das 1.4-3.6-Dianhydro-hexitol gewonnen wird oder es wird nach Neutralisation des im Reaktionsgemisch befindlichen Halogenids mit Basen, wie z. B. Natriumhydroxid oder Triethylamin, entweder extraktiv oder destillativ aufgearbeitet. Nach der Umsetzung des Hexitols mit Chlorwasserstoff kann alles aktive Chlorid (HCl bzw. Säurechlorid) mit anorganischen Basen wie z. B. NaOH, KOH, $Ca(OH)_2$, $Ba(OH)_2$ oder organischen Basen wie Trialkylaminen, Pyridinen etc. neutralisiert werden. Die anschließend extraktiv oder bevorzugt destillativ isolierten Dianhydro-hexitole werden in hoher Ausbeute und guter Reinheit in kristalliner Form erhalten.

Die so gewonnenen 1.4-3.6-Dianhydro-hexitole sind wertvolle Bausteine für das Diisocyanat-Polyadditionsverfahren, brauchbar als Starter für Polyether, z. B. durch Addition von Ethylen- und/oder Propylenoxid, als Bausteine für Polyester, als Ausgangspolyol für Polycarbonate, sowie als Härter für Epoxidharze. In den nachfolgenden Beispielen werden die eingesetzten Verbindungen in Gew.-Teilen angegeben.

Beispiel 1

400 Teile D-Mannit werden mit 20 Teilen Essigsäure auf 120 bis 140 °C erwärmt und in die Mischung Chlorwasserstoff eingeleitet, wobei von außen nicht weitergeheizt wird. Nach beendeter Gasaufnahme (ca. 150 Minuten) wird bei der erreichten Endtemperatur von etwa 140 °C 30 Minuten weitergerührt, wobei gleichzeitig Wasser und Essigsäure abdestilliert werden. Anschließend wird der Rückstand im Vakuum fraktioniert. Das Isomannid (1.4-3.6-Dianhydro-D-mannit) destilliert im Wasserstrahlvakuum bei 155 bis 165 °C/15 bis 18 bar, bzw. im Ölpumpenvakuum bei 145 °C/0,15 bar und wird mit einer Ausbeute von 65 % der Theorie und einem Schmelzpunkt von 83 bis 85 °C erhalten. Zum Vergleich Literaturausbeute 2) : 36 % der Theorie.

Beispiel 2

400 Teile D-Mannit und 20 Teile Acetylchlorid werden in einem Druckreaktor bei Raumtemperatur mit 40 bar Chlorwasserstoff beaufschlagt und anschließend auf 50 °C gebracht, wobei der Druck durch Nachpressen von Chlorwasserstoff auf 40 bar gehalten wird. Nachdem kein Druckabfall mehr eintritt (ca. 110 Minuten) wird noch 30 Minuten der Druck gehalten, weitergerührt und anschließend bei Raumtemperatur entspannt und im Vakuum fraktioniert. Ausbeute an Isomannid : 80 bis 85 % der Theorie.

Beispiel 3

364 Teile D-Sorbit werden mit 20 Teilen Eisessig bei 45 °C 40 Stunden mit 40 bar Chlorwasserstoff in einem Druckgefäß beaufschlagt. Anschließend wird das Reaktionsgemisch fraktioniert. Nachdem Wasser und Essigsäure bei Normaldruck abdestilliert sind, gewinnt man bei 165 bis 175 °C/17 bis 18 bar 247 Teile (84,5 % der Theorie) an 1.4-3.6-Dianhydro-sorbit als Öl, das alsbald kristallisiert und einen Schmelzpunkt von 54,5 bis 55,5 °C aufweist.

Beispiel 4

Allgemeine Vorschrift.

400 g Hexitol werden mit 5 Mol-% einer organischen Carbonsäure, oder eines organischen Säureanhydrids oder eines organischen Carbonsäurehalogenids in einem Druckreaktor bei Raumtemperatur mit 80 g Chlorwasserstoff versetzt und anschließend auf 100 °C gebracht.

Nach 3 Stunden wird bei Raumtemperatur entspannt und das Reaktionsgemisch bei 20 bis 40 °C mit halbkonzentrierter Natronlauge gegen Bromphenolblau-Indikator solange neutralisiert, bis kein Farbumschlag von blau nach gelb mehr erfolgt. Anschließend wird fraktioniert destilliert oder mit Essigester erschöpfend extrahiert. Die extraktiv gewonnene Produktlösung wird eingeengt, wobei das Produkt anfällt und gegebenenfalls im Vakuum destilliert wird. Ausbeuten zwischen 70 bis 85 % der Theorie werden üblicherweise erzielt.

**Ansprüche**

1. Verfahren zur Herstellung von 1.4-3.6-Dianhydro-hexitolen aus Hexitolen durch Wasserabspaltung mittels starker Säuren, dadurch gekennzeichnet, daß man als saures Dehydratisierungsreagens gasförmigen Halogenwasserstoff und gegebenenfalls organische Carbonsäuren, Carbonsäurehalogenide und/-oder Carbonanhydride verwendet und die Reaktion in Abwesenheit von Wasser und organischen Lösungsmitteln durchführt.

2. Verfahren nach Anspruch 1, dadurch gekennzeichnet, daß man Hexitole als Feststoff, als Flüssigkeit oder als Schmelze, gegebenenfalls in Gegenwart von organischen Carbonsäuren, Carbonsäurechloriden und symmetrischen sowie unsymmetrischen Carbonsäureanhydriden in einem Verhältnis von 0,01 bis 600 Mol-%, bezogen auf Hexitole, mit gasförmigem Chlorwasserstoff in 0,01- bis 1 000-fach äquivalenten Verhältnis, bevorzugt bis zur Sättigung, versetzt.

3. Verfahren nach Ansprüchen 1 und 2, dadurch gekennzeichnet, daß die Reaktionspartner bei einer Temperatur von 0 bis 300 °C und einem Druck von 1 bis 250 bar zur Reaktion gebracht werden.

4. Verfahren nach Ansprüchen 1 bis 3, dadurch gekennzeichnet, daß man die organischen Carbonsäuren, Carbonsäurechloride und symmetrischen sowie unsymmetrischen Carbonsäureanhydride in einem Verhältnis von 0,01 bis 10 Mol-%, bezogen auf Hexitol, verwendet und die Reaktionspartner bei einer Temperatur von 20 bis 160 °C und einem Druck von 1 bis 40 bar zur Reaktion bringt.

5. Verfahren nach Ansprüchen 1 bis 4, dadurch gekennzeichnet, daß man Chlorwasserstoff bis zur Sättigung in das Reaktionsgemisch einleitet.

6. Verfahren gemäß Ansprüchen 1 bis 5, dadurch gekennzeichnet, daß Carbonsäuren der allgemeinen Formel

$$R\text{—}(ROOH)_x$$

mit

x = 1 bis 6 und

R = x-wertiger Rest einer aliphatischen gesättigten oder ungesättigten, einer aromatischen oder araliphatischen Carbonsäure,

Carbonsäurehalogenide der allgemeinen Formel

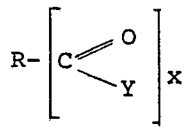

wobei

Y = Halogen und

x und R die oben angegebene Bedeutung haben,

und/oder symmetrische oder unsymmetrische Carbonsäureanhydride der allgemeinen Formel

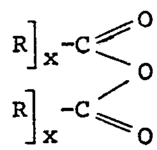

wobei

R = gleiche oder verschiedene Reste der oben angeführten Bedeutung sind,

verwendet werden.

7. Verfahren nach Ansprüchen 1 bis 6, dadurch gekennzeichnet, daß nach Umsetzung der Hexitole mit Chlorwasserstoff das Isohexitol durch fraktionierte Destillation im Vakuum isoliert wird.

8. Verfahren nach Ansprüchen 1 bis 7, dadurch gekennzeichnet, daß nach Umsetzung des Hexitols mit Chlorwasserstoff alles aktive Chlorid mit anorganischen oder organischen Basen neutralisiert und anschließend das Dianhydro-hexitol extraktiv und/oder destillativ isoliert wird.

**Claims**

1. Process for the preparation of 1,4-3,6-dianhydro-hexitols from hexitols by the elimination of water by means of strong acids, characterised in that gaseous hydrogen halide is used as the acid dehydrating reagent and organic carboxylic acids, carboxylic acid halides and/or carboxylic acid anhydrides are optionally used and the reaction is carried out in the absence of water and organic solvents.

2. Process according to Claim 1, characterised in that hexitols, in the form of a solid, a liquid or a melt, are treated with gaseous hydrogen chloride in 0.01 to 1,000 times the equivalent proportion, preferably up to the saturation point, optionally in the presence of organic carboxylic acids, carboxylic acid chlorides and symmetric or asymmetric carboxylic acid anhydrides in a proportion of 0.01 to 600 mol%, based on the hexitols.

3. Process according to Claims 1 and 2, characterised in that the reactants are reacted at a temperature of 0 to 300 °C and a pressure of 1 to 250 bars.

4. Process according to Claims 1 to 3, characterised in that the organic carboxylic acids, carboxylic acid chlorides and symmetric or asymmetric carboxylic acid anhydrides are used in a proportion of 0.01 to 10 mol%, based on hexitol, and the reactants are reacted at a temperature of 20 to 160 °C and under a pressure of 1 to 40 bars.

5. Process according to Claims 1 to 4, characterised in that hydrogen chloride is introduced into the reaction mixture until saturation is reached.

6. Process according to Claims 1 to 5, characterised in that carboxylic acids of the general formula

$$R\text{—}(ROOH)_x$$

in which

X = 1 to 6 and

R = an x-valent radical of an aliphatic saturated or unsaturated carboxylic acid or of an aromatic or araliphatic carboxylic acid,

carboxylic acid halides of the general formula

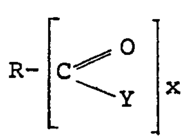

wherein
Y = halogen and
x and R have the above-mentioned meaning,
and/or symmetric or asymmetric carboxylic acid anhydrides of the general formula

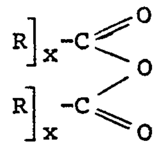

wherein
R = identical or different radicals of the above-mentioned meaning,
are used.

7. Process according to Claims 1 to 6, characterised in that after the reaction of the hexitols with hydrogen chloride the isohexitol is isolated by fractional distillation in vacuo.

8. Process according to Claims 1 to 7, characterised in that after the reaction of the hexitol with hydrogen chloride all the active chloride is neutralised with inorganic or organic bases and the dianhydro-hexitol is subsequently isolated by extraction and/or distillation.

**Revendications**

1. Procédé de préparation de 1.4-3.6-dianhydro-hexitols à partir d'hexitols par séparation d'eau au moyen d'acides forts, caractérisé en ce que, comme réactif acide de déshydratation, on utilise un halogénure d'hydrogène gazeux et éventuellement des anhydrides d'acides carboxyliques, des halogénures d'acides carboxyliques et/ou des acides carboxyliques organiques et l'on effectue la réaction en absence d'eau et de solvants organiques.

2. Procédé suivant la revendication 1, caractérisé en ce que, aux hexitols sous forme d'une matière solide, d'un liquide ou d'une masse fondue, éventuellement en présence d'anhydrides d'acides carboxyliques symétriques et asymétriques, de chlorures d'acides carboxyliques et d'acides carboxyliques organiques dans un rapport de 0,01 à 600 % molaires, calculé sur les hexitols, on ajoute du chlorure d'hydrogène gazeux dans un rapport équivalent de 0,01 à 1 000 fois, de préférence, jusqu'à saturation.

3. Procédé suivant les revendications 1 et 2, caractérisé en ce qu'on fait réagir les partenaires réactionnels à une température de 0 à 300 °C et sous une pression de 1 à 250 bars.

4. Procédé suivant les revendications 1 à 3, caractérisé en ce qu'on utilise les anhydrides d'acides carboxyliques symétriques et asymétriques, les chlorures d'acides carboxyliques et les acides carboxyliques organiques dans un rapport de 0,01 à 10 % molaires, calculé sur l'hexitol et l'on fait réagir les partenaires réactionnels à une température de 20 à 160 °C et sous une pression de 1 à 40 bars.

5. Procédé suivant les revendications 1 à 4, caractérisé en ce qu'on introduit du chlorure d'hydrogène dans le mélange réactionnel jusqu'à saturation.

6. Procédé suivant les revendications 1 à 5, caractérisé en ce qu'on utilise des acides carboxyliques de formule générale

$$R\text{—}(ROOH)_x$$

où
x = 1 à 6 et
R = radical x-valent d'un acide carboxylique aliphatique saturé ou insaturé, d'un acide carboxylique aromatique ou d'un acide carboxylique araliphatique,
des halogénures d'acides carboxyliques de formule générale

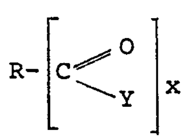

où
Y = halogène et
x et R ont les signification indiquées ci-dessus,
et/ou des anhydrides d'acides carboxyliques symétriques ou asymétriques de formule générale

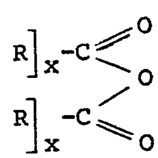

où

R = radicaux identiques ou différents de la signification indiquée ci-dessus.

7. Procédé suivant les revendications 1 à 6, caractérisé en ce que, après la réaction des hexitols avec le chlorure d'hydrogène, on isole l'isohexitol par distillation fractionnée sous vide.

8. Procédé suivant les revendications 1 à 7, caractérisé en ce que, après la réaction de l'hexitol avec le chlorure d'hydrogène, on neutralise tout le chlorure actif avec des bases inorganiques ou organiques, puis on isole le dianhydro-hexitol par extraction et/ou par distillation.